(19) **Europäisches Patentamt / European Patent Office / Office européen des brevets**

(11) **EP 2 191 810 B1**

(12) **EUROPEAN PATENT SPECIFICATION**

(45) Date of publication and mention
of the grant of the patent:
**03.01.2018 Bulletin 2018/01**

(21) Application number: **08765004.0**

(22) Date of filing: **03.06.2008**

(51) Int Cl.:
*A61F 13/02* (2006.01)    *A61K 9/70* (2006.01)
*A61K 47/32* (2006.01)    *A61K 47/34* (2017.01)
*A61L 15/58* (2006.01)    *B32B 25/08* (2006.01)
*C09J 7/02* (0000.00)    *A61K 8/02* (2006.01)
*A61Q 19/08* (2006.01)

(86) International application number:
**PCT/JP2008/060191**

(87) International publication number:
**WO 2009/041121 (02.04.2009 Gazette 2009/14)**

(54) **COSMETIC AUXILIARY PATCH MATERIAL AND METHOD OF COSMETIZING THEREWITH**

KOSMETISCHES HILFSPFLASTERMATERIAL UND VERFAHREN ZUR KOSMETISCHEN ANWENDUNG

MATÉRIAU DE TIMBRE AUXILIAIRE COSMÉTIQUE ET PROCÉDÉ DE COSMÉTISATION AVEC CELUI-CI

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **28.09.2007 JP 2007256570**

(43) Date of publication of application:
**02.06.2010 Bulletin 2010/22**

(73) Proprietors:
• **Nichiban Co. Ltd.**
 **Tokyo 112-8663 (JP)**
• **Kazki International Corporation**
 **Shinjuku-ku,**
 **Tokyo 1600017 (JP)**

(72) Inventors:
• **UCHIDA, Kazuko**
 **Tokyo 160-0017 (JP)**
• **WATANABE, Shuichi**
 **Tokyo 112-8663 (JP)**
• **TAKAGI, Yasuyo**
 **Tokyo 112-8663 (JP)**
• **KANESHIGE, Mami**
 **Tokyo 112-8663 (JP)**

• **FUKANO, Kenji**
 **Tokyo 112-8663 (JP)**
• **FUJISAWA, Hiromichi**
 **Tokyo 112-8663 (JP)**

(74) Representative: **Gill Jennings & Every LLP**
 **The Broadgate Tower**
 **20 Primrose Street**
 **London EC2A 2ES (GB)**

(56) References cited:
 **EP-A2- 1 025 864    JP-A- 10 023 923**
 **JP-A- 2001 278 739    JP-A- 2001 299 808**
 **JP-A- 2005 089 438    JP-A- 2007 021 068**
 **JP-A- 2007 314 584**

• **DATABASE WPI Week 199846 Thomson Scientific, London, GB; AN 1998-535395 XP002728506, & JP H10 234772 A (MAKINAE N) 8 September 1998 (1998-09-08)**
• **DATABASE WPI Week 200630 Thomson Scientific, London, GB; AN 2006-288932 XP002728507, & JP 2006 104174 A (LION CORP) 20 April 2006 (2006-04-20)**

**Description**

TECHNICAL FIELD

The present invention relates to a makeup-assisting patch and a makeup method using the makeup-assisting patch. More particularly, the present invention relates to a makeup-assisting patch suitable for use in a makeup method intended for rehabilitation, to which attention is paid as a new field of makeup methods, and a makeup method using the makeup-assisting patch.

BACKGROUND ART

In general, a person can become heightened in spirit and feel affluent by adjusting the appearance thereof beautifully. Even a person having no affliction, to say nothing of a person afflicted with the appearance of one's face or body, can become heightened in spirit and feel affluent by adjusting the appearance thereof beautifully by makeup. Accordingly, the makeup is an important matter for a person to live a life in society. The makeup may also be said to be a work for a person to derive its own spirits. Therefore, the makeup has been applied with various cosmetic compositions from ancient times.

It is an important subject for a person having the skin (hereinafter referred to as "skin with damage or the like") with wrinkles, pouches, spots, bruises, freckles, pores, wound scars, acne spots, burn scars, discoloration by a dermal disease, and/or the like in particular from the viewpoint of rehabilitation to make the skin with damage or the like inconspicuous.

The rehabilitation means various kinds of physiotherapy that are performed for rehabilitating a person damaged in a bodily function to a life in society. Attention is paid to rehabilitation by psychotherapy in addition to such physiotherapy intended for recovering the bodily function or in place of the physiotherapy. However, in a person damaged in the face or body thereof, or a person afflicted with the appearance of one's face or body, the result satisfactory enough to live an ordinary life in society is often not achieved by only the rehabilitation by the conventional physiotherapy and/or the psychotherapy.

There has been proposed a makeup method that makeup variously devised is applied to a person having the skin with damage or the like, thereby making the damage or the like inconspicuous to adjust the appearance thereof beautifully. According to this makeup method, the person having the skin with damage or the like can be enrolled in or rehabilitated to a life in society without feeling psychological pressure or having inferiority complex. A typical makeup method exhibiting an effect as rehabilitation is habitually taken on a worldwide scale in new terms of REHABILIMAKE (trademark).

This makeup method also has meaning of a makeup technique acquired by a person having damage or the like on the face or body thereof for being enrolled in or rehabilitated to a life in society like the ordinary rehabilitation. This makeup method can also exhibit advantage as the rehabilitation by the psychotherapy. However, this method can exhibit an epoch-making psychological effect to a person to be applied in that specific improvement in appearance is made beyond the level of the mere psychotherapy.

The makeup method intended for rehabilitation makes the damage or the like on the skin inconspicuous to adjust the appearance beautifully. However, a principal object thereof is not to hide the damage or the like on the skin. This makeup method is different from an ordinary makeup method in that the principal object thereof is to create a positive psychological state capable of receiving the final one's own appearance through makeup in order for a person having damage on the face or body thereof to be enrolled in or rehabilitated to a life in society. According to the makeup method exhibiting the effect as the rehabilitation, the quality of a life of the person having damage on the face or body thereof can be enhanced.

In the makeup method combined with rehabilitation, makeup techniques using various cosmetic compositions have been developed. However, in some cases, a sufficient effect may not be exhibited by only application of the cosmetic compositions according to the degree of damage or the like on the face or body. Even in case of intending to recover the damage on the skin as the rehabilitation, there may be a limit to the makeup method by only the application of the cosmetic compositions.

Specifically, it may be difficult according to, for example, the degree of damage or the like in some cases to adjust the appearance by only the makeup method using the cosmetic compositions. The site of the damage or the like on the skin may be easily poisoned with the cosmetic compositions. The site of the damage or the like on the skin may be exposed to ultraviolet rays of sunlight to cause skin roughness or inhibit the damage or the like from being recovered. It is difficult to adjust the skin with deep wrinkles or furrows beautifully by only the application of the cosmetic compositions.

There has heretofore been proposed a method of applying a certain patch (hereinafter referred to as "a makeup-assisting patch") to the skin in addition to the makeup method using the cosmetic compositions. Japanese Utility Model Registration No. 3116047 (Patent Art. 1) has proposed a foundation seal obtained by placing a pressure sensitive adhesive on one side of a stretchable film and placing a cosmetic material on the other side. This foundation seal is used by applying it to a diseased part for making discoloration or local discoloration such as spots or freckles inconspicuous.

The fact that wrinkles or pouches are formed on the skin at the corners of the eyes, lower portions of the eyes, the mouth, cheeks or the like with aging is a factor of bringing affliction into persons. Japanese Patent Application Laid-Open No. 10-194962 (Patent Art. 2) has proposed a wrinkle-removing patch comprising a transparent tape having adhesion property on a back surface thereof. This wrinkle-removing patch is used for preventing wrinkling or removing wrinkles by applying it to a lower portion of an eye to hold a wrinkled site in a pressed state for a long period of time.

Japanese Patent Application Laid-Open No. 2004-313277 (Patent Art. 3) has proposed a stretchable tape for cosmetic surgery obtained by applying a pressure sensitive adhesive thickly to a stretchable base. This stretchable tape is used for ironing and stretching wrinkles on the surface of the skin by sticking it on the surface of the skin to fix them.

Japanese Patent Application Laid-Open No. 10-234772 (Patent Art. 4) has proposed an adhesive bandage for hiding a wound, which is obtained by embossing a portion coming into contact with the wound for hiding a wound scar left on the surface of a body. A microfilm (Patent Art. 5) of Japanese Utility Model Application No. 55-1747 (Japanese Utility Model Application Laid-Open No. 56-104519) has proposed a bruise-hiding film obtained by applying an adhesive to a complexioned artificial film having water resistance and making a great number of fine vents in the film.

However, the makeup-assisting patches heretofore proposed have involved such problems that (1) the patch itself in a state stuck on the skin is conspicuous and thus unnatural, (2) a person to be applied has a feeling of physical disorder in a stuck state, (3) a stuck portion is different in the touch from the healthy skin around the stuck portion, (4) a rash is easily caused at the stuck site, (5) it is difficult to apply cosmetic compositions to the surface of the skin including the surface of the patch stuck, and (6) a function of protecting a site having damage or the like on the skin is insufficient.

The principal object of the conventional makeup-assisting patches is to hide damage or the like on the skin or stretch wrinkles, and so such patches do not exhibit a sufficient function from the viewpoint of rehabilitation. Therefore, the conventional makeup-assisting patches have been not sufficient in devices for components making up the patches, such as a base and a pressure sensitive adhesive.

In general, a patch comprises of a base layer composed of a plastic film, paper, fabric or the like, and a pressure sensitive adhesive layer provided on one surface of the base layer. If the base layer is opaque, has a color tone markedly different from the color of the skin or is too thick, the stuck state itself of the resulting patch is conspicuous to fail to achieve the object of makeup assistance.

In most of the conventional makeup-assisting patches, the thickness of the base layer is 20 to 30 $\mu$m, and the thickness of the pressure sensitive adhesive layer is also relatively great, so that a stuck portion is easily conspicuous for the skin having fine and shallow skin furrows like in the forearm or the face, or such a patch gives a user a feeling of physical disorder during sticking. Thus, these patches have been not satisfactory. Such makeup-assisting patches are not conformable to wrinkles and skin furrows. The conventional wrinkle-removing patches are suitable for use during sleeping or in household, but not suitable for going out in a stuck state.

If the flexibility of the base layer is insufficient, the resulting makeup-assisting patch cannot easily follow the movement of the skin surface and gives a person to be applied a feeling of physical disorder or gives a third party a feeling of unnatural. The pressure sensitive adhesive layer easily causes skin roughness such as rash because it comes into direct contact with the skin surface. The makeup-assisting patch is required, as an important characteristic, not to cause skin roughness such as rash because it is often stuck over a long period of time.

If the adhesive strength of the pressure sensitive adhesive layer is too weak, it is difficult to keep the stuck state over a long period of time. If the adhesive strength of the pressure sensitive adhesive layer is too weak, the patch is simply peeled upon face washing or the like. If the adhesive strength of the pressure sensitive adhesive layer is too strong, peeling of the patch becomes difficult, or pain is felt upon peeling of the patch. On the other hand, the makeup-assisting patch is also required, as another important characteristic, to enable it to be formed into a shape simply separable as needed.

If the water vapor permeability of the makeup-assisting patch is too low, sweat accumulates on the skin surface of a stuck portion to cause a rash or inhibit the protecting effect on a wounded portion. If the back surface (surface provided with no pressure sensitive adhesive layer) of the base layer is too smooth, or too strong in hydrophobicity, it is difficult to apply a makeup method of applying a cosmetic composition from above the makeup-assisting patch in a stuck state. In order to apply the cosmetic composition, it is necessary for the base layer to have moderate water absorption property and be easily intimate with water. From the viewpoint of rehabilitation, a function of protecting the skin with damage or the like from cosmetic compositions and ultraviolet rays may be required in some cases. However, the conventional makeup-assisting patches have been insufficient in such a protecting function.

As described above, the conventional makeup-assisting patches have been insufficient in any required property or function for applying them to a makeup method combined with rehabilitation. Therefore, there is a demand for a new makeup-assisting patch capable of synthetically solving the above-described various problems.

Patent Art. 1: Japanese Utility Model Registration No. 3116047

Patent Art. 2: Japanese Patent Application Laid-Open No. 10-194962

Patent Art. 3: Japanese Patent Application Laid-Open No. 2004-313277

Patent Art. 4: Japanese Patent Application Laid-Open No. 10-234772

Patent Art. 5: Microfilm of Japanese Utility Model Application No. 55-1747 (Japanese Utility Model Application Laid-Open No. 56-104519)

DISCLOSURE OF THE INVENTION

PROBLEMS SOUGHT FOR SOLUTION BY THE INVENTION

It is an object of the present invention to provide a makeup-assisting patch suitable for use in makeup intended for rehabilitation.

Specifically, the object of the present invention is to provide a makeup-assisting patch suitable for sticking on the skin with apparent damage or the like, such as wrinkles, pouches, spots, bruises, freckles, pores, wound scars, acne spots, burn scars or discoloration by a dermal disease.

More specifically, the object of the present invention is to provide a makeup-assisting patch which is inconspicuous in a stuck state, does not give a user a feeling of physical disorder, can follow the movement of the skin, does not cause a rash, has a function of protecting a wounded portion, has the same touch as the healthy skin around it, is easy to apply a cosmetic composition thereto and permits natural makeup.

Another object of the present invention is to provide a makeup method using the makeup-assisting patch having such excellent various characteristics, for example, a makeup method, in which the makeup-assisting patch according to the present invention is stuck on the skin with apparent damage or the like, such as wrinkles, pouches, spots, bruises, freckles, pores, wound scars, acne spots, burn scars or discoloration by a dermal disease, and makeup is applied thereto, thereby hiding such a portion to make that portion the same appearance as the healthy skin around that portion so as to make inconspicuous, or in which the makeup-assisting patch according to the present invention is stuck so as to make wrinkles on the face at the corners of the eyes, lower portions of the eyes, the mouth, cheeks or the like with aging inconspicuous.

The present inventors have carried out an extensive investigation with a view toward achieving the above-described objects. As a result, the inventors have reached a patch, in which (1) an extremely thin polyurethane elastomer layer is used as a base layer, (2) a surface (back surface) of the base layer, which is provided with no pressure sensitive adhesive layer, is properly embossed, (3) a thin acrylic pressure sensitive adhesive layer composed of a copolymer containing an alkyl (meth)acrylate unit having an alkyl group having 8 to 12 carbon atoms in a proportion of 70% by weight or more is used as a pressure sensitive adhesive layer, and (4) the total thickness of the layers is extremely thin.

The patch of the present invention is extremely thin in the thickness of each layer and the total thickness of the layers, is excellent in conformability to wrinkles and fine furrows of the skin and in the following ability to the movement of the skin, has water vapor permeability, is free of a feeling of physical disorder and occurrence of a rash upon sticking, has a protecting function against cosmetic compositions and ultraviolet rays, has the same touch as the healthy skin around it, is easy to apply a cosmetic composition thereto, and can exhibit the combination of excellent various characteristics as a makeup-assisting patch. The present invention has been led to completion on the basis of these findings.

MEANS FOR THE SOLUTION OF THE PROBLEMS

According to the present invention, there is provided a makeup-assisting patch having a layer structure that a pressure sensitive adhesive layer is provided on one surface of a base layer, wherein (1) the base layer is a layer of a polyether type polyurethane elastomer having a glass transition temperature within the range of -10 to -70°C, (2) the pressure sensitive adhesive layer is an acrylic pressure sensitive adhesive layer composed of a copolymer containing at least one monomer unit selected from the group consisting of an alkyl acrylate and an alkyl methacrylate each having an alkyl group having 8 to 12 carbon atoms in a proportion of 70% by weight or more, and (3) the thickness of the base layer is 1 to 8 $\mu$m, the thickness of the pressure sensitive adhesive layer is 1 to 8 $\mu$m, and the total thickness of these both layers is 2 to 15 $\mu$m, (4) the pressure sensitive adhesive layer has a peeling force within a range of from 0.1 to 3 N/10 mm when a peeling force is measured at a crosshead speed of 300±30 mm/min in accordance with Japanese Industrial Standard JIS Z 0237, (5) the makeup-assisting patch has a tensile load within a range of from 0.01 to 1.2 N/cm in both machine and transverse directions when a 10% tensile load is measured in accordance with Japanese Industrial Standard JIS Z 0237, (6) the makeup-assisting patch has a water vapor transmission rate of 1,000 g/m$^2$·24 hr or more when a water vapor transmission rate is measured under conditions of a temperature of 40°C and a relative humidity of 90% in accordance with Japanese Industrial Standard JIS Z 0208, and (7) the base layer is such that a surface provided with no pressure sensitive adhesive layer is subjected to embossing.

According to the present invention, there is also provided a makeup method comprising sticking the above-described makeup-assisting patch on the skin with the pressure sensitive adhesive layer thereof.

EFFECTS OF THE INVENTION

According to the present invention, there is provided a makeup-assisting patch which is conformable to wrinkles and fine irregularities (fine furrows) of the skin, is inconspicuous at a stuck portion, can easily follow the movement of the skin, hardly causes a rash, protects the skin from cosmetic compositions and ultraviolet rays, has the same touch as the healthy skin around it, and permits applying a cosmetic composition in a stuck state. The makeup-assisting patch according to the present invention is suitable for use as an auxiliary material in a makeup method intended for rehabilitation. The makeup-assisting patch according to the present invention can prevent the skin from directly applying a cosmetic composition thereto by applying the cosmetic composition on to the makeup-assisting patch stuck on the skin and permits removing the cosmetic composition together with the patch by simply peeling the patch, and thus has advantages from the viewpoints of simplicity and influence on the skin.

BRIEF DESCRIPTION OF THE DRAWINGS

[FIG. 1] schematically illustrates an example of a makeup-assisting patch in the form of a drop having a projected portion.
[FIG. 2] schematically illustrates an example of a makeup-assisting patch in the form of a triangle.
[FIG. 3] illustrates a specific example of a cosmetic method using the makeup-assisting patch in the form of the drop having the projected portion.

BEST MODE FOR CARRYING OUT THE INVENTION

1. Base layer:

The base layer in the makeup-assisting patch of the present invention is formed from a thin layer of a polyurethane elastomer. The polyurethane elastomer is an elastomer having a urethane group in its molecule and is formed by a polyaddition reaction of a polyol component and a diisocyanate component. A long-chain diol is used as the polyol component. However, a short-chain diol may be used in combination as a chain-lengthening agent in addition to the long-chain diol. Besides, a crosslinking agent such as a monomolecular polyol such as trimethylolpropane, glycerol or sorbitol may be used. A production technique of the polyurethane elastomer is a technique well-known in the art.

Since the nature of the polyurethane elastomer is greatly affected by the kind of the polyol component making up a soft segment, the elastomer is classified according to the kind of the polyol component. Specifically, the polyurethane elastomer is divided roughly into (1) a caprolactone type polyurethane elastomer synthesized by a polyaddition reaction of a polylactone ester polyol obtained by ring-opening polymerization of caprolactone, and a diisocyanate (not according to the invention), (2) an adipic acid ester type polyurethane elastomer synthesized by a polyaddition reaction of an adipic acid ester polyol of adipic acid and a glycol, and a diisocyanate (not according to the invention), and (3) a polyether type polyurethane elastomer, according to the invention, synthesized by a polyaddition reaction of a polyether polyol such as polytetramethylene glycol obtained by ring-opening polymerization of tetrahydrofuran, and a diisocyanate.

The polyurethane elastomer is excellent in mechanical strength such as tensile strength and tear strength, abrasion resistance, low-temperature properties and flexibility, and moreover a thin film thereof is excellent in ultraviolet light absorption property, water absorption property and water vapor permeability. A makeup-assisting patch using a thin film of the polyurethane elastomer as a base layer is well conformable to wrinkles and fine irregularities such as furrows of the skin and does not give a user a feeling of physical disorder when stuck on the skin.

In the makeup-assisting patch using the thin film of the polyurethane elastomer as the base layer, accumulation of sweat or occurrence of stuffiness at a stuck site of the skin is slight because the base layer has both water absorption property and water vapor permeability. Therefore, the makeup-assisting patch according to the present invention is extremely little in occurrence of a rash or itch at the stuck site. The thin film of the polyurethane elastomer has water absorption property and is easily intimate with water, so that a cosmetic composition is easily applied thereto. The polyether type polyurethane elastomer exhibits an excellent synthetic function as a base layer of a makeup-assisting patch intended for rehabilitation.

Examples of the diisocyanate include aromatic diisocyanates such as tolylene diisocyanate, diphenylmethane diisocyanate, polymethylenepolyphenylene polyisocyanate, tolidine diisocyanate and naphthalene diisocyanate; and aliphatic diisocyanates such as hexamethylene diisocyanate, isophorone diisocyanate, xylylene diisocyanate, dicyclohexylmethane diisocyanate and hydrogenated xylylene diisocyanate.

In the caprolactone type polyurethane elastomer, a polylactone ester polyol obtained by ring-opening polymerization of caprolactone is used as a polyol component. In the adipic acid ester type polyurethane elastomer, an adipic acid ester polyol of adipic acid and a glycol is used as a polyol component. In the polyether type polyurethane elastomer of the invention, a bifunctional polyether polyol such as poly(oxypropylene) glycol (PPG) or poly(oxytetramethylene) glycol (PTMG) is used as a polyol component.

As Examples of commercially available products of the polyether type polyurethane elastomer for use in the present invention, may be mentioned ELASTRAN (trademark) (1180A, 1190ATR, 1195ATR, 1198ATR, 1154D, 1164D, ET385, ET880, ET885, ET890, ET858D, ET860D, ET864D, NY90A, NY97A, ET379) available from BASF Japan Ltd., MIRACTRAN (trademark) (E300 series, P300 series) available from Nippon Miractoran Co., Ltd., REZAMIN (trademark) (P-2000 series) available from Dainichiseika Color & Chemicals Mfg. Co., Ltd., and PANDEX (trademark) (T8175, T8180, T8185, T8190, T8195, DP9370A, 5377A, 588, KU2-8659, DP5094A) available from DIC Bayer Polymer Ltd.

The glass transition temperature (Tg) of the polyurethane elastomer used in the present invention is within the range of -10°C to -70°C. The glass transition temperature can be measured by means of a differential scanning calorimeter (DSC). The makeup-assisting patch according to the present invention is used by sticking it on the surface of the skin having an average bodily temperature of 36.5°C. The glass transition temperature of the base layer is low, whereby the flexibility of the makeup-assisting patch during sticking can be ensured. When the base layer of the makeup-assisting patch during sticking is excellent in flexibility, a cosmetic composition is easily applied thereto (good in spreading of a cosmetic composition). When water is applied to the surface of the base layer of the makeup-assisting patch during sticking before a cosmetic composition is applied, the cosmetic composition is more easily applied thereto. When water is applied from the back surface of the base layer upon application of a cosmetic composition, the base layer composed of the polyurethane elastomer becomes soft, and so such a patch is easier to become conformable to the surface of the skin, and spreading of a cosmetic composition thereon becomes better. Since the polyurethane elastomer is low in glass transition temperature, it is excellent in flexibility at a temperature near to a bodily temperature and is hence excellent in conformability to the skin surface and applicability of cosmetic compositions.

The thickness of the base layer is within a range of from 1 to 8 $\mu$m, more preferably from 1 to 6 $\mu$m, still more preferably from 1 to 5 $\mu$m. When the thickness of the base layer falls within this range, the resulting makeup-assisting patch conforms to fine irregularities of the skin, easily follows the movement of the skin and makes a stuck portion inconspicuous. If the thickness of the base layer is less than 1 $\mu$m, the strength of the base layer becomes insufficient, and the base layer may be cut in some cases during sticking of the resulting makeup-assisting patch on the skin or upon peeling thereof from the skin. If the thickness of the base layer exceeds 10 $\mu$m on the other hand, a stuck portion becomes conspicuous, and moreover the conformability to the skin surface and following ability to the movement of the skin of the resulting patch are lowered.

The thickness of the base layer is extremely thin, whereby water vapor permeability can also be improved. An extremely thin polyurethane elastomer layer is excellent in water absorption property and water vapor permeability, so that it is extremely little to cause a rash or itch or inhibit the protection of a wounded portion by accumulation of sweat or water at a stuck portion of the makeup-assisting patch.

In the makeup-assisting patch according to the present invention, the base layer composed of the polyurethane elastomer has a thin thickness and is excellent in flexibility, so that when a skin site with wrinkles or pouches is stretched to stick the patch thereon, the patch closely adheres to the skin, and becomes a state entered into the interiors of skin furrows or wrinkles when the stretched state is released. Therefore, the makeup-assisting patch becomes a state as if the patch has been folded at the surface of the skin with wrinkles or the like, and so the stuck site looks flat. As a result, the patch can exhibit an effect that wrinkles or pouches look lessened.

The back surface (surface provided with no pressure sensitive adhesive layer) of the base layer is subjected to embossing. The embossing can be conducted by causing a polyurethane elastomer film to pass through between embossing rolls. When the polyurethane elastomer layer is formed by a solution casting process, the embossing can be conducted by a process of casting a solution on a carrier layer (a support to be coated with the solution), on the surface of which fine irregularities have been provided. The fine irregularities on the surface of the carrier layer are transferred to the polyurethane elastomer layer. Accordingly, the embossing in the present invention also includes any other process capable of forming fine irregularities on the surface of the polyurethane elastomer layer in addition to a mechanical work using the embossing rolls.

The degree of the embossing work can be quantitatively evaluated by a gloss of the back surface of the base layer as measured at an angle of 60°. According to Japanese Industrial Standard (JIS), as the standard of the gloss, a reflectance of 10% as measured at an incident angle of 60° on the surface of glass having a refractive index of 1.567 is regarded as a gloss of 100%. In the present invention, MICRO-TRI-GROSS (manufactured by Toyo Seiki Co.) is used as a glossmeter to measure a gloss at an angle of 60°.

The gloss of the back surface of the base layer used in the present invention is within a range of from 0.5 to 7.5, preferably from 1 to 6. The base layer composed of the polyurethane elastomer is embossed in such a manner that the gloss of the base layer falls within the above range, whereby the stuck state of the resulting makeup-assisting patch can be prevented from giving an apparently unnatural feeling, and moreover a cosmetic composition is easier to be applied to the patch.

In the makeup-assisting patch according to the present invention, the material of the base layer is the polyether type polyurethane elastomer, whereby the patch has the same touch as the healthy skin. The skin with a wound scar or keloid loses the texture of the healthy skin and is often smooth and slippery like the surface of a plastic film. According to the kind of a dermal disease, excessive irregularities, texture disorder, drying and/or the like may be caused on the skin in some cases to be dry and rough to the touch. When the makeup-assisting patch according to the present invention is stuck on such skin site with the wound scar or the like as described above, the same touch as the healthy skin around the patch can be given. The back surface of the base layer of the makeup-assisting patch is embossed, whereby the same touch as the healthy skin around the patch can be more improved. The improvement in the touch can more enhance such a psychological rehabilitation effect that a feeling of satisfaction in mind of a person to be applied is improved.

The polyurethane elastomer can be formed into a thin base layer by adopting a general film or sheet forming process, for example, an extrusion process, solution casting process or calendering process. The solution casting process, in which a solution of the polyurethane elastomer in an organic solvent is cast on a support, and the organic solvent is volatilized off, is a preferable film-forming process in that a uniform and thin polyurethane elastomer layer can be easily formed. When fine irregularities are provided on the surface of the support, the fine irregularities can be transferred to the surface of the polyurethane elastomer layer formed. When a carrier material is used as the support, a makeup-assisting patch having a carrier layer on the back surface of the support layer composed of the polyurethane elastomer is obtained.

The base layer preferably scarcely has a directional difference (anisotropy) of physical properties from the viewpoint of a feeling of physical disorder (a feeling of resistance of the makeup-assisting patch felt at the time the skin has extended) during sticking. From this point of view, a solution casting process or calendering process, by which a directional difference of physical properties does not appear, is preferred as the film forming process, with the solution casting process being more preferred.

A colorant such as a pigment or dye, and various kinds of additives such as an antioxidant, an ultraviolet absorbent, a lubricant, a filler and a softener may be contained in the polyurethane elastomer as needed. The colorant can be utilized to adjust the color tone of the makeup-assisting patch to the color of the skin, and a colorant of a yellow or orange is preferred as the colorant for making the appearance of a site stuck on the skin with damage or the like inconspicuous.

2. Pressure sensitive adhesive layer:

In a patch provided with a pressure sensitive adhesive layer, various kinds of pressure sensitive adhesives such as rubbery pressure sensitive adhesives, acrylic pressure sensitive adhesives and silicone pressure sensitive adhesives are generally used as a pressure sensitive adhesive. The pressure sensitive adhesive layer of the present invention is formed by using an acrylic pressure sensitive adhesive from the viewpoints of hardly causing a rash upon sticking, giving a feeling of transparence and easily adjusting adhesion properties.

As the acrylic pressure sensitive adhesive, is used a copolymer (hereinafter may also be referred to as "acrylate copolymer") containing at least one monomer unit selected from the group consisting of an alkyl acrylate and an alkyl methacrylate each having an alkyl group having 8 to 12 carbon atoms in a proportion of 70% by weight or more.

Among the monomers, an alkyl acrylate is preferred, and more preferable specific examples of the acrylate monomer include 2-ethylhexyl acrylate, isooctyl acrylate, n-octyl acrylate and isononyl acrylate.

In the acrylate copolymer used in the present invention, the content of the alkyl acrylate and/or the alkyl methacrylate unit having an alkyl group having 8 to 12 carbon atoms is preferably 70% by weight or more, more preferably 80% by weight or more. The acrylate copolymer used in the present invention may be a copolymer of 2 or more monomers of the above-mentioned monomers. However, a copolymer with any other comonomer is more preferred.

Examples of the comonomer include functional group-containing monomers such as monomers having a carboxyl group, such as acrylic acid and methacrylic acid; and monomers having a hydroxyl group, such as hydroxyethyl acrylate and hydroxypropyl acrylate. As other comonomers, may be used various monomers such as vinyl acetate, styrene, vinylpyrrolidone and acrylamide. An alkyl acrylate or alkyl methacrylate having another alkyl group than the alkyl group having 8 to 12 carbon atoms, such as ethyl acrylate or butyl acrylate, may also be used as a comonomer.

As preferable examples of the acrylate copolymer, may be mentioned copolymers obtained by copolymerizing 70 to 95% by weight of at least one monomer selected from the group consisting of an alkyl acrylate and an alkyl methacrylate each having an alkyl group having 8 to 12 carbon atoms, 1 to 10% by weight of the functional group-containing comonomer and 0 to 25% by weight of any other comonomer.

The acrylic pressure sensitive adhesive may be prepared by subjecting the above-described monomer components to solution polymerization in an organic solvent such as toluene, hexane or ethyl acetate under a nitrogen atmosphere using a peroxide such as benzoyl peroxide or an azo compound such as AIBN (azobisisobutyronitrile) as an initiator. The acrylic pressure sensitive adhesive may also be prepared by emulsifying and dispersing the monomer components in water and then conducting emulsion polymerization. A crosslinking agent such as an epoxy resin may also be added to the acrylic pressure sensitive adhesive to crosslink the adhesive at a step of forming the pressure sensitive adhesive layer.

The thickness of the pressure sensitive adhesive layer is 1 to 8 $\mu$m, particularly preferably 1 to 6 $\mu$m. The pressure sensitive adhesive layer is small in resistance to stretching and high in stretchability compared with the base layer, so that the thickness range thereof may be more widened than the thickness range of the base layer. However, if the thickness of the pressure sensitive adhesive layer is too great, there is a tendency for the resulting patch to become difficult to conform to wrinkles or fine furrows of the skin, and a stuck state becomes easily conspicuous. If the thickness of the pressure sensitive adhesive layer is too thin, there is a tendency for formation of a uniform pressure sensitive adhesive layer to become difficult, and moreover, the sticking ability of the resulting patch on the skin becomes easily insufficient.

When the pressure sensitive adhesive layer is extremely thinned, the resulting patch becomes excellent in conformability to fine irregularities of the skin like skin furrows that are micro-furrows appeared on the skin, but the adhesive strength thereof to the skin may be lowered. In the present invention, the adhesion property of the pressure sensitive adhesive is preferably controlled in such a manner that in a makeup-assisting patch, the total thickness of the base layer and the pressure sensitive adhesive layer is within a range of from 2 to 15 $\mu$m, the pressure sensitive adhesive layer shows 90-degree peeling force of 0.1 N/10 mm or more to a Bakelite plate as measured at a crosshead speed of 300$\pm$30 mm/min in accordance with JIS Z 0237. This peeling force is property corresponding to the adhesive strength of the pressure sensitive adhesive layer.

Although the lower limit value of the peeling force (adhesive strength) of 0.1 N/10 mm is not always a large value, a sufficient function as a makeup-assisting patch can preferably be exhibited so far as the thickness of the base layer and the peeling force satisfy the respective proper ranges because the base layer is thin and flexible. The peeling force of the pressure sensitive adhesive layer is 0.1 to 3 N/10 mm, more preferably 0.2 to 3 N/10 mm. If the peeling force (adhesive strength) is too high, a rash is easy to occur at a stuck portion, and difficulty is easily encountered on peeling of the makeup-assisting patch from the skin after use.

Various kinds of additives such as a colorant such as a pigment or a dye may be added to the pressure sensitive adhesive layer as needed. The colorant can be utilized to adjust the color tone of the makeup-assisting patch to the color of the skin, and a colorant with yellow or orange tone is preferred as the colorant for making the appearance of a site stuck on the skin with damage or the like inconspicuous. Besides, for example, an antibacterial agent, a moisturizer, a bleaching agent, an anti-wrinkle agent, an anti-sagging agent, a dullness-proofing agent, a hair restorer, a nail beautifier, vitamins and perfumes may also be incorporated to impart additional functions to the patch.

3. Makeup-assisting patch

The makeup-assisting patch according to the present invention has a layer structure that a pressure sensitive adhesive layer is provided on one surface of a base layer.
The thickness of the base layer is 1 to 8 $\mu$m, more preferably 1 to 6 $\mu$m, still more preferably 1 to 5 $\mu$m. The thickness of the pressure sensitive adhesive layer is 1 to 8 $\mu$m, still more preferably 1 to 6 $\mu$m. The total thickness of the base layer and the pressure sensitive adhesive layer is within a range of from 2 to 15 $\mu$m, more preferably from 2 to 10

When the makeup-assisting patch according to the present invention has additional layers such as a carrier layer and a separator layer, the thickness thereof comes to be added. Since these additional layers are peeled upon use, the thickness of the makeup-assisting patch in a stuck state is a total thickness of the thickness of the base layer and the thickness of the pressure sensitive adhesive layer.

The thickness of the makeup-assisting patch is 2 to 15 $\mu$m, and the thickness of the base layer is 1 to 8 $\mu$m, and the thickness of the pressure sensitive adhesive layer is 1 to 8 $\mu$m. When the thickness of the makeup-assisting patch is 2 to 10 $\mu$m, it is preferable that the thickness of the base layer is 1 to 6 $\mu$m, and the thickness of the pressure sensitive adhesive layer is 1 to 8 $\mu$m. When the thickness of the makeup-assisting patch is 2 to 7 $\mu$m, it is preferable that the thickness of the base layer is 1 to 5 $\mu$m, and the thickness of the pressure sensitive adhesive layer is 1 to 6 $\mu$m. The thickness of each of the base layer, the pressure sensitive adhesive layer and the makeup-assisting patch can be measured by a dial gauge.

In the makeup-assisting patch according to the present invention, the base layer is a polyether type polyurethane elastomer layer, the pressure sensitive adhesive layer is an acrylic pressure sensitive adhesive layer and more the thicknesses of the base layer and the pressure sensitive adhesive layer and the total thickness of these both layers fall within the above-described respective ranges, whereby the makeup-assisting patch can exhibit an excellent synthetic function.

The makeup-assisting patch according to the present invention is stuck on the skin (skin with damage or the like) with wrinkles, pouches, spots, bruises, freckles, pores, wound scars, acne spots, burn scars, discoloration by a dermal disease, and/or the like, and a cosmetic composition is applied on to the patch as needed, whereby the site of the damage or the like can be hided and made inconspicuous by bringing the same appearance as the healthy skin around it. The application of the cosmetic composition means an ordinary makeup treatment that various kinds of cosmetic compositions are applied to and spread on the skin including the makeup-assisting patch.

The makeup-assisting patch according to the present invention can conform to fine irregularities (fine furrows) of the skin, makes a stuck portion inconspicuous,
can easily follow the movement of the skin, is hard to cause a rash, protects the skin from cosmetic compositions and ultraviolet rays and permits applying cosmetic compositions thereto in a stuck state. The conformation to the fine irregularities of the skin means that the makeup-assisting patch according to the present invention enters the fine irregularities of the skin, including skin furrows, to exhibit the same appearance as the skin. When the back surface of the base layer in the makeup-assisting patch according to the present invention is embossed, a slight difference in thickness is made on the base layer to exhibit better flexibility, whereby it is easy for the patch to enter the skin furrows and wrinkles.

Since the pressure sensitive adhesive layer in the makeup-assisting patch according to the present invention is thin, and the water absorption property and water vapor permeability of the base layer, it is little to cause a rash or itch by accumulation of sweat or occurrence of stuffiness. When water is applied to the back surface of the base layer upon application of a cosmetic composition, the base layer composed of the polyurethane elastomer becomes soft, and so such a patch is easier to become conformable to the surface of the skin, and spreading of the cosmetic composition thereon becomes better. Since the glass transition temperature of the polyurethane elastomer is low, it is excellent in flexibility at a temperature near to a bodily temperature and is hence excellent in conformability to the skin surface and applicability of cosmetic compositions.

The base layer composed of the polyurethane elastomer is excellent in ultraviolet light absorption property. The makeup-assisting patch according to the present invention exhibits an ultraviolet transmittance of 20% of less, preferably 15% or less in a wavelength range of from 280 to 400 nm as measured within this wavelength range. In a wavelength range of from 280 to 360 nm, the patch exhibits an ultraviolet transmittance of 14% or less. In a wavelength range of 300 nm or less in particular, the ultraviolet transmittance is greatly lowered to 10% or less. The ultraviolet transmittance was measured by means of an ultraviolet-visible spectrophotometer (Ubest-V530) manufactured by JASCO Corporation.

When the makeup-assisting patch according to the present invention is stuck on a wounded site of the skin, the wounded site can be protected from irradiation of ultraviolet rays because the makeup-assisting patch according to the present invention is excellent in ultraviolet light absorption property. When the makeup-assisting patch according to the present invention is stuck on the wounded site of the skin, and a cosmetic composition is applied thereto, there is no possibility that a rash or the like may be caused by the cosmetic composition because the wounded site comes into no direct contact with the cosmetic composition. Accordingly, the makeup-assisting patch according to the present invention has a function of protecting the surface of the skin with damage or the like in a makeup method intended for rehabilitation using the makeup-assisting patch.

The makeup-assisting patch according to the present invention can be used in stretching wrinkles or pouches formed on the skin at the corners of the eyes, lower portions of the eyes, the mouth, cheeks or the like with aging and making them inconspicuous. Such use is also a part of the makeup method using the makeup-assisting patch of the present invention. When a site of the skin with wrinkles or pouches is stretched, and the makeup-assisting patch according to the present invention is stuck thereon in a state that the wrinkles or pouches have been stretched, the makeup-assisting patch of the present invention closely adheres to this site. When the stretched state is released, it is presumed that the makeup-assisting patch becomes a state entered into the interiors of skin furrows or wrinkles. As a result, the makeup-assisting patch exists in such a state as folded at the surface of this skin, and the whole appearance looks flat. It is presumed that such appearance that wrinkles or pouches are lessened is formed by simply sticking the makeup-assisting patch according to the present invention on the site with wrinkles or pouches. When water is applied on to the base layer after sticking, the base layer swells by water absorption, so that the effect of stretching wrinkles or pouches is more improved. Since the base layer composed of the polyurethane elastomer has water absorption property, the base layer absorbs water volatilized out of the body and swells with time without applying water thereto after sticking, so that it is expected that the effect of stretching wrinkles or pouches is increased. The swelling degree of this base layer composed of the polyurethane elastomer is determined by immersing a polyurethane elastomer having a thickness of 75 $\mu$m and a size of 100 mm x 100 mm for 24 hours in water, measuring lengths before and after the immersion and calculating out (Increment of the length)/(100 mm). The swelling degree is 1 to 20%, preferably 2 to 10%.

The makeup-assisting patch according to the present invention has a feature that it follows the movement of the skin and does not give a user a feeling of physical disorder during sticking. This feature relates to the degree of ease of elongation in the makeup-assisting patch. A 10% tensile load corresponding to the resistance to stretching of the makeup-assisting patch may be used as an index to this ease of elongation.

Specifically, the makeup-assisting patch according to the present invention exhibits a tensile load within a range of from 0.01 to 1.2 N/cm in both machine and transverse directions when a 10% tensile load is measured in accordance with Japanese Industrial Standard JIS Z 0237. This tensile load is 0.01 to 1.2 N/cm, more preferably 0.01 to 1.0 N/cm, still more preferably 0.01 to 0.5 N/m, particularly preferably 0.01 to 0.3 N/cm in both machine and transverse directions.

In order to make the makeup-assisting patch according to the present invention inconspicuous and lessening a feeling of physical disorder, it has been found

that the thickness of the makeup-assisting patch, the thickness of the base layer and the ease of elongation of the makeup-assisting patch preferably reside in a specific relationship. For example, if the thickness of the makeup-assisting patch or the base layer is thick, the following ability to the movement of the human skin is insufficient. On the other hand, if the makeup-assisting patch is easy to be elongated, its following ability to the movement of the skin is good, so that the thickness of the makeup-assisting patch or the base layer may be somewhat thick. Thus, the thickness of the makeup-assisting patch or the base layer and the ease of elongation correlate to each other.

The makeup-assisting patch according to the present invention exhibits a water vapor transmission rate of 1,000 g/m$^2$·24 hr or more when measured under conditions of a temperature of 40°C and a relative humidity of 90% in accordance with Japanese Industrial Standard JIS Z 0208. This water vapor transmission rate is preferably 3,000 to 10,000 g/m$^2$·24 hr.

The intended water vapor transmission rate can be achieved by selecting the kinds and the thicknesses of the base layer and the pressure sensitive adhesive layer making up the makeup-assisting patch, and the like. Besides, a method of enhancing the water vapor transmission rate by making fine holes in the makeup-assisting patch may also be adopted.

In the makeup-assisting patch according to the present invention, the base layer or the pressure sensitive adhesive layer or these both layers are preferably colored yellow with a colorant. The layers are preferably colored yellow with the colorant in such a manner that the measured values of a CIE L*a*b*(CIELAB) colorimetric system by a colorimeter satisfy the relationship indicated by the following expressions 1 to 3:

$$45 < L* < 90 \quad \cdots \quad 1$$

$$-8 < a* < 12 \quad \cdots \quad 2$$

$$20 < b* < 50 \quad \cdots \quad 3$$

The measured values of the CIELAB colorimetric system by the colorimeter preferably satisfy the relationship among 55 < L* < 80, -4 < a* < 8 and 30 < b* < 50.

In the CIELAB colorimetric system, the L* value indicates the degree of lightness. The a* value indicates the degree of a red color when the numerical value becomes high or the degree of a green color when the numerical value becomes low. The b* value indicates the degree of a yellow color when the numerical value becomes high or the degree of a blue color when the numerical value becomes low. The measurement of the colorimetric system is conducted by a method comprising using a colorimeter (CM-3500d, manufactured by MINOLTA), determining a reference L*a*b* by a standard white plate and indicating a surface color by the CIELAB colorimetric system.

In order to color each layer of the makeup-assisting patch according to the present invention, it is only necessary to use pigments or dyes of respective colors such as yellow, cyan, magenta and black either singly or in combination of 2 or more colorants thereof so as to give a desired color tone. These colorants may be used by mixing them with each layer. Alternatively, a method of coloring the base layer by applying a coating liquid containing these colorants to the base layer may also be adopted. In order to achieve the above-mentioned measured values of the CIELAB colorimetric system, for example, a method using a mixture of a cyan colorant 3, a magenta colorant 3, and a yellow colorant 47 (weight ratio) is mentioned. Such a mixture may also be prepared by mixing inks of the respective colors.

In the makeup-assisting patch according to the present invention, the base layer or the pressure sensitive adhesive layer or these both layers may be colored yellow or orange. The layers are colored into the color tone of yellow (also including orange) with a colorant in such a manner that the measured values of the CIELAB colorimetric system by the colorimeter satisfy the relationship indicated by the above-mentioned expressions 1 to 3, whereby the appearance of a stuck site of the skin with damage or the like becomes inconspicuous, and moreover makeup by application of a cosmetic composition becomes easy. In general, a color tone of yellow is preferred. However, a color tone of orange is also preferred for a blue bruise or the like.

The makeup-assisting patch according to the present invention has a layer structure comprising the base layer and the pressure sensitive adhesive layer. However, its handling may be difficult in some cases because the thicknesses of both layers are thin. In order to improve the handling ability of the makeup-assisting patch according to the present invention, it is preferable that a carrier layer is arranged on the surface of the base layer, and the separator layer is arranged on the surface of the pressure sensitive adhesive layer to provide a makeup-assisting patch having a 4 layer structure of carrier layer/base layer/pressure sensitive adhesive layer/separator layer. Another elastomer layer excellent in adhesion to the base layer and the pressure sensitive adhesive layer may be arranged between both layers, or another pressure sensitive adhesive layer may also be arranged between the carrier layer and the base layer.

The makeup-assisting patch according to the present invention is provided as a multilayer structure of the 4-layer structure, whereby its handling ability can be improved. In the makeup-assisting patch having this multilayer structure, the carrier layer can be used as a support layer, and the base layer composed of the polyurethane elastomer can be formed thereon by the solution casting process. Since this makeup-assisting patch has the separator layer, it can be divided into individual products to produce, sell and use them.

The carrier layer is arranged in the makeup-assisting patch according to the present invention, thereby permitting preventing the base layer from wrinkling, strengthening the stiffness of the makeup-assisting patch and making a sticking operation on a sticking site easy.

The carrier layer is preferably formed by using a film composed of any one of various kinds of thermoplastic resins, for example, polyurethane, polyethylene, polypropylene, ionomers, polyamide, polyvinyl chloride, ethylene-vinyl acetate copolymers, thermoplastic polyesters and polytetrafluoroethylene. Aiming at environmental protection, the carrier layer may also be formed by using a film composed of any one of various kinds of plastics having biodegradability, typified by polyhydroxybutyrates, polyhydroxybutyrate resins, polyhydroxyalkanoates, maltotriose, polylactic acid, polylactic acid resins, polyethylene succinate resins, polybutylene succinate resins, polycaprolactone resins, poly(butylene adipate-co-terephthalate), poly(tetramethylene adipate-co-terephthalate), poly(ethylene terephthalate) resins, polyvinyl alcohol, polyglycolic acid, starch fatty acid esters, starch-processed resins, starch polyesters, cellulose acetate and chitosan. The various kinds of films may be in a state laminated on paper. These carrier layers desirably have a thicker thickness or stronger stiffness compared with the polyurethane elastomer layer.

In the makeup-assisting patch according to the present invention, the separator layer is preferably provided from the viewpoint of protecting the pressure sensitive adhesive layer till just before sticking. As the separator layer, is used one generally called lubricant paper, release paper, release liner or the like in a technical field of pressure sensitive adhesive tapes. Examples of the separator layer include polyethylene terephthalate films, the surfaces of which have been subjected to a silicone treatment, and laminates of polyethylene, the surface of which has been subjected to a silicone treatment, and paper.

4. Production process of makeup-assisting patch

The makeup-assisting patch according to the present invention is preferably produced by a production process comprising forming a pressure sensitive adhesive layer on a separator layer, forming a polyurethane elastomer layer of a base layer on a carrier layer and then laminating the pressure sensitive adhesive layer on the separator layer and the polyurethane elastomer layer on the carrier layer on each other. The makeup-assisting patch according to the present invention may also be produced by a process comprising a step of laminating a carrier layer and a polyurethane elastomer film on each other.

In the present invention, fine irregularities are formed on the back surface of the base layer of the makeup-assisting patch by embossing. When the process of forming the polyurethane elastomer layer of the base layer on the carrier layer is adopted, fine irregularities are provided on the surface of a carrier material forming the carrier layer in advance, and the fine irregularities are transferred to the base layer composed of the polyurethane elastomer.

In order to form the fine irregularities on the carrier material, a publicly known embossing process using embossing rolls can be adopted. As another process, the surface of the carrier material may be subjected to a sandblasting treatment, or a filler may be contained in the carrier material, thereby forming fine irregularities.

In order to form the pressure sensitive adhesive layer on the separator layer, a process of coating a separator material with a solution of an acrylic pressure sensitive adhesive by a solution casting process and drying the solution is preferably adopted.

5. Makeup method

In order to apply makeup with the makeup-assisting patch according to the present invention, a method comprising sticking the makeup-assisting patch on the skin with the pressure sensitive adhesive layer thereof is adopted.

After sticking, various kinds of cosmetic compositions are applied if desired. In other words, after the makeup-assisting patch is stuck on the skin, and a cosmetic composition is further applied on to the surface of the skin, including the surface of the base layer of the makeup-assisting patch stuck. A process, in which the makeup-assisting patch is stuck on the skin, water is applied to the surface of the base layer of the makeup-assisting patch stuck to make the surface conformable to water, and the cosmetic composition is then further applied on to the surface of the skin, including the surface of the base layer, is preferably adopted.

The makeup-assisting patch according to the present invention is stuck on a wrinkled portion of the skin in a state that wrinkles have been stretched, whereby such appearance that the wrinkles are lessened can be formed. The makeup-assisting patch according to the present invention is applied to the wrinkled portion over a long period of time, whereby it can also be expected that wrinkles are stretched and lessened. Even in the case where pouches are present on the skin, the makeup-assisting patch according to the present invention is used, whereby the appearance can be adjusted beautifully to make the pouches inconspicuous.

In the makeup method intended for rehabilitation, a makeup method, in which the makeup-assisting patch according to the present invention is stuck on the skin (skin with damage or the like) with spots, bruises, freckles, pores, wound scars, acne spots, burn scars, discoloration by a dermal disease, or the like, and a cosmetic composition is applied on to the patch, is adopted, whereby the site of the skin with damage or the like can be made the same appearance as the healthy skin around that site so as to make inconspicuous. When the makeup-assisting patch according to the present invention is stuck, the touch of the site with damage or the like can be made the same as the healthy skin.

As an example of a makeup method of making the site with damage or the like of the skin inconspicuous, is mentioned a makeup method according to the following steps of procedure.

(1) The skin is fully massaged with an oil (for example, squalane oil) and water.
(2) The makeup-assisting patch according to the present invention is stuck on the skin.
(3) A milky foundation with yellow tone is spread as a makeup primer on the skin including the portion, on which the makeup-assisting patch has been stuck, with fingers.
(4) A pasty foundation with yellow tone having high covering power (high in concealing property and covering property) and a solid foundation with dark brown tone are mixed and applied on to the milky foundation.
(5) A powder is thinly applied thereon (applied so as to be lightly pressed).

The makeup method is not limited to the method according to the above-described steps. For example, when damage or discoloration of the skin is excessive, the steps of (4) and (5) are repeated to apply the pasty foundation and the solid foundation one over the other several times like steps of (1), (2), (3), (4), (5), (4), and (5), whereby the concealing property and covering property can also be enhanced. When the damage or the like of the skin is light, a part of the steps may be omitted.

The foundation with dark brown tone in the step (4) has an effect of making spots inconspicuous by matching the color tone with the spots to utilize an optical illusion. The foundation with dark brown tone is applied to side surfaces of a face, whereby the face looks a centripetal face (a tightened steric face).

In the step (4), a solid foundation with orange tone may also be additionally mixed and applied as needed according to the color tone of a bruise or the like of the skin in addition to the solid foundations with yellow tone and dark brown tones.

In the case of the above-described makeup method, when water is applied to the surface of the base layer of the makeup-assisting patch after sticking of the makeup-assisting patch to make the surface conformable to water, spreading of cosmetic compositions becomes better. As a specific method for making the surface conformable to water, water or toilet water is sprayed on the surface of the base layer after the makeup-assisting patch is stuck, or the surface of the base layer is wetted with water or toilet water by hand. After the base layer is then pressed for from several seconds to about 1 minute with the palm of a hand to make the surface conformable to water, makeup is applied. Tap water or mineral water may be used as the water.

The above-described makeup method can exhibit a particularly marked effect when the polyurethane elastomer forming the base layer is a polyether type polyurethane elastomer. The makeup-assisting patch is preferably colored yellow or orange because the concealing property is more improved. The makeup-assisting patch according to the present invention can prevent the skin from directly applying a cosmetic composition thereto by applying the cosmetic composition on to the makeup-assisting patch stuck on the skin and permits removing the cosmetic composition together with the patch by simply peeling the patch, and thus has advantages from the viewpoints of simplicity and influence on the skin.

The makeup-assisting patch according to the present invention can be used in stretching wrinkles or pouches formed on the skin at the corners of the eyes, lower portions of the eyes, the mouth, cheeks or the like with aging and making them inconspicuous. In order to achieve this effect of making wrinkles or pouches inconspicuous, the separator layer is first peeled when the makeup-assisting patch according to the present invention is actually stuck on the skin to stick the patch on the skin at the wrinkled or pouched portion with the surface of the pressure sensitive adhesive layer, and the carrier layer is then peeled to leave only a makeup-assisting patch composed of the base layer and the pressure sensitive adhesive layer on the wrinkled or pouched portion.

The makeup-assisting patch is stuck on a wrinkled portion of the skin with the surface of the pressure sensitive adhesive layer in a state that wrinkles have been stretched. In this method, the makeup-assisting patch with the carrier layer is stuck in a state that the skin has been stretched and become flat free of wrinkles, and the carrier layer is then peeled, so that it is presumed that the wrinkles are returned to original in a state that the carrier layer has been peeled, and the stretching has been released, and at the same time the makeup-assisting patch closely adhered to the skin enters the wrinkles in a form as if folded. As a result, the skin is in such a state that the wrinkles are filled with the makeup-assisting patch, and the wrinkles look lessened. When water is applied on to the base layer, it is expected that the makeup-assisting patch absorbs water and swells to increase the wrinkle-stretching effect. Even when the makeup-assisting patch according to the present invention is applied to the skin with a pouched site, it is expected that an excellent pouch-stretching effect is exhibited.

When the makeup-assisting patch according to the present invention is stuck, there is also an effect of stretching the skin to make wrinkles or pouches inconspicuous and making a face line neat. The makeup-assisting patch may also be stuck on the skin by pulling the skin with a wrinkled or pouched portion by hand so as to move to another portion.

The form and size of the makeup-assisting patch according to the present invention may be suitably set in accordance with the form and size of the skin with damage or the like. Examples of the form of the makeup-assisting patch include rectangles, squares, triangles, other polygons, circles, crescents, ellipses and drops. The makeup-assisting patch according to the present invention may also be used by cutting a free size tape or sheet into a piece of desired form and size by scissors or the like.

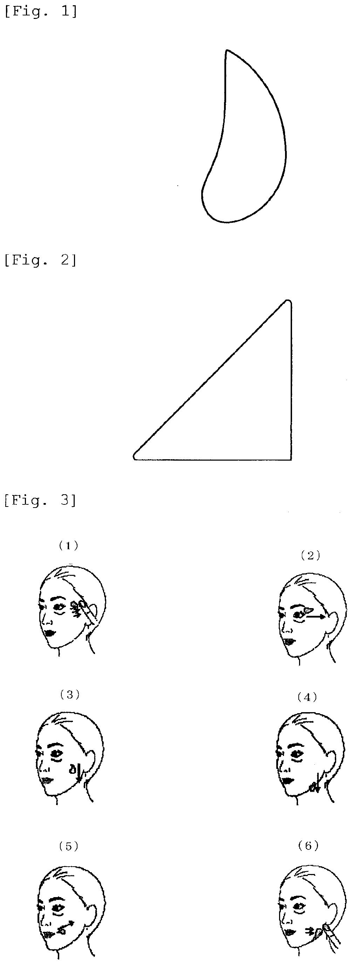

In such a manner, the makeup-assisting patch according to the present invention may be formed into various forms in accordance with the form of the damage or the like. At this time, the patch is preferably die-cut into a form having a projected portion for giving a role of a finger grip. As the form having the projected portion, a drop form (tear drop form) is preferred from the viewpoints of ease of use and general-purpose properties. An example of the drop form is illustrated in FIG. 1. This form is a drop form having a projected portion and in a form that the whole thereof is curved like a saddle put on a riding horse for making it easy to follow a curved site such as a face or neck. When the separator layer is peeled, a nail is put on the projected portion in case of the drop form to easily peel the separator layer. In this case, the projected portion also fulfills a role of a finger grip. An example of a makeup-assisting patch, the die-cut piece of which is in the form of a triangle, is illustrated in FIG. 2. Each top of the triangle fulfills a role of a finger grip as a projected portion. The triangle is preferably a right-angled triangle.

The makeup-assisting patch according to the present invention is preferably a tape or sheet and die-cut into a form having a projected portion. A tape or sheet may also be die-cut into a large number of pieces each having a projected portion, and the pieces may be taken out one by one upon use. The form having the projected portion is preferably a drop form having a length ranging from 0.5 to 6 cm and a width ranging from 0.3 to 3 cm, or a triangle form, each side of which has a length ranging from 0.5 to 8 cm.

An example of steps of procedure for sticking a makeup-assisting patch, the die-cut piece of which is in the form of a drop (tear drop) having a projected portion, is illustrated in FIG. 3.

(1) A method of pulling up the pouched eyes or dropped eyelids, or making wrinkles at the corners of the eyes inconspicuous ··· FIG. 3(1)

The patch is stuck while obliquely pulling up the skin at the temple with a finger.

(2) A method of lengthening the eyes ··· FIG. 3(2)

The patch is stuck while stretching the skin in a direction of an arrow with a finger. It is effective to perform this method after the step (1).

(3) An improvement in conspicuousness of cheekbones ··· FIG. 3(3)

The patch is stuck while stretching the skin on the cheekbone in a downward direction.

(4) An improvement in a face line (contours of the face including jaws or cheeks) or a loose double chin ··· FIG. 3(4)

The patch is stuck while pulling the skin on the chin by a finger so as to move downward.

(5) Pulling up the corners of the mouth (a person hard to make a smiling face by a disease such as facial paralysis or depression) ··· FIG. 3(5)

The patch is stuck while pulling the corner of the mouth in a lateral direction as illustrated in the drawing.

(6) A method of making nasolabial folds (two skin folds formed with aging, which run from each side of the nose to the corners of the mouth) inconspicuous ··· FIG. 3(6)

The patch is stuck while drawing the skin up to under each ear in a direction of an arrow.

EXAMPLES

The present invention will hereinafter be described more specifically by Examples. However, the present invention is not limited to these examples, and may be variously applied within limits not departing from the technical idea thereof. The evaluating methods are as described above. However, some measuring methods thereof are described below again.

(1) Gloss:

As the standard of gloss according to Japanese Industrial Standard (JIS), a reflectance of 10% as measured at an incident angle of 60° on the surface of glass having a refractive index of 1.567 is regarded as a gloss of 100%. MICRO-TRI-GROSS (manufactured by Toyo Seiki Co.) was used as a glossmeter to measure a gloss at an angle of 60°.

(2) Ultraviolet transmittance:

An ultraviolet transmittance was measured at a measuring wavelength of 280 to 400 nm by means of an ultraviolet-visible spectrophotometer (Ubest-V530 Model, manufactured by JASCO Corporation).

(3) 10% tensile load:

A 10% tensile load was measured on the basis of JIS Z 0237. Specifically, a makeup-assisting patch was stretched by 10% by an Instron type tensile tester to measure a stress (N) at that time. The resultant value was converted to a value in a 10 mm width. The measurement was conducted in both machine and transverse directions. However, the measured values were almost the same, and so the measured value in the machine direction is shown.

(4) Adhesive strength:

Adhesive strength was measured at a crosshead speed of $300\pm30$ mm/min in accordance with JIS Z 0237. Specifically, 90 degree peeling force of a 10 mm wide makeup-assisting patch stuck on a Bakelite plate was measured by an Instron type tensile tester.

(5) Water vapor transmission rate:

A water vapor transmission rate of a makeup-assisting patch was measured under conditions of a temperature of 40°C and a relative humidity of 90% in accordance with JIS Z 0208.

(6) Glass transition temperature:

A glass transition temperature of a polyurethane elastomer was measured by means of a differential scanning calorimeter (manufactured by Shimadzu Corporation).

(7) Evaluation of inconspicuousness:

A patch obtained by cutting in a size of 17 mm x 29 mm was used to visually make evaluation of inconspicuousness. The patch was stuck on a cheek of an adult woman. The stuck state on the subject was visually evaluated by 5 adult women and ranked as A where 3 women or more judged it hard to be conspicuous, B where 2 women judged it hard to be conspicuous, or C where one woman or less judged it hard to be conspicuous.

(8) Evaluation of a feeling of physical disorder:

A patch was stuck on the cheek of each of 5 adult women to be ranked as AA where 4 women or more did not feel a feeling of physical disorder, A where 3 women did not feel a feeling of physical disorder, B where 2 women did not feel a feeling of physical disorder, or C where one woman or less did not feel a feeling of physical disorder.

[Example 1]

An organic solvent solution of an acrylic pressure sensitive adhesive (a copolymer of 2-ethyl-hexyl acrylate/vinyl acetate/acrylic acid = 85/11/4 % by weight) was applied to one surface of a separator layer (release paper) by a bar coating method so as to give a dry coating thickness of 5 μm and then dried to form a pressure sensitive adhesive layer.

On the other hand, woodfree paper, the polypropylene surface of which had been embossed, was used as a carrier layer, and a solution of a polyether type polyurethane elastomer was applied thereto by a bar coating method so as to give a dry coating thickness of 5 μm and dried to form a base layer. The polyether type polyurethane elastomer is ELASTRAN (trademark) ET880 (glass transition temperature: -45°C) available from BASF Japan Ltd.

A layer material of 75 μm in thickness and 100 mm x 100 mm in size was produced with this polyurethane elastomer, and immersed for 24 hours in water to measure a swelling degree. As a result, it was 5%.

The base layer obtained above was laminated on the pressure sensitive adhesive layer to produce a makeup-assisting patch of a 4-layer structure of carrier layer/base layer/pressure sensitive adhesive layer/separator layer.

The gloss of the thus-obtained makeup-assisting patch was 5.1, the adhesive strength was 0.58 N/10 mm, and the 10% tensile load was 0.14 N/10 mm. The water vapor transmission rate of this makeup-assisting patch was 3,290 g/ m$^2$·24 hr as measured at a temperature of 40°C and a relative humidity of 90% on the basis of JIS Z 0208, and the patch had sufficient gas permeability. The ultraviolet transmittance of this makeup-assisting patch was 14.5% in a wavelength range of from 280 to 400 nm. The patch was evaluated as to inconspicuousness and a feeling of physical disorder. The results are shown collectively in Table 1.

[Example 2] [not according to the invention]

A makeup-assisting patch was produced in the same manner as in Example 1 except that the solution of the polyether type polyurethane elastomer in Example 1 was changed to a solution of a polyester type polyurethane elastomer (ELASTRAN ET680 available from BASF Japan Ltd., glass transition temperature: -35°C). The patch was evaluated as to inconspicuousness and a feeling of physical disorder. The results are shown collectively in Table 1.

[Comparative Example 1]

The polyether type polyurethane elastomer and acrylic pressure sensitive adhesive used in Example 1 were used to produce a makeup-assisting patch, in which the thicknesses of the base layer and the pressure sensitive adhesive layer after drying were 12 μm and 3 μm, respectively. The patch was evaluated as to inconspicuousness and a feeling of physical disorder. The results are shown collectively in Table 1.

[Comparative Example 2]

The polyether type polyurethane elastomer and acrylic pressure sensitive adhesive used in Example 1 were used to produce a makeup-assisting patch, in which the thicknesses of the base layer and the pressure sensitive adhesive layer after drying were 3 μm and 18 μm, respectively. The patch was evaluated as to inconspicuousness and a feeling of physical disorder. The results are shown collectively in Table 1.

[Comparative Example 3]

The polyether type polyurethane elastomer layer in Example 1 was changed to a polyethylene terephthalate layer (LUMIRROR available from Toray Industries, Inc., glass transition temperature: 75°C), and the acrylic pressure sensitive adhesive was used to produce a makeup-assisting patch, in which the thicknesses of the base layer and the pressure sensitive adhesive layer after drying were 5 μm and 5 μm, respectively. The patch was evaluated as to inconspicuousness and a feeling of physical disorder. The results are shown collectively in Table 1.

[Table 1]

|  | Inconspicuousness | Feeling of physical disorder |
|---|---|---|
| Example 1 | A | AA |
| Example 2 | A | AA |
| Comp. Example 1 | C | B |
| Comp. Example 2 | B | B |
| Comp. Example 3 | C | C |

[Consideration]

The results of Examples 1 and 2 were good in both evaluations of inconspicuousness and a feeling of physical disorder felt by the subjects. On the other hand, when the base layer is thick (Comparative Example 1), the thickness of the pressure sensitive adhesive layer and the total thickness of the base layer and the pressure sensitive adhesive layer are thick (Comparative Example 2), or the glass transition temperature of the base layer is high (Comparative Example 3), the resulting patch is such that a stuck portion is conspicuous, or a feeling of physical disorder is felt.

<Evaluation of makeup>

Each of the makeup-assisting patches in Examples 1 and 2 was die-cut into a 40-mm square, the separator layer was peeled to stick it on woodfree paper with the surface of the pressure sensitive adhesive layer, and the carrier layer was peeled.

(a) Case of using no water:

A solid foundation (powder) was applied on to the base layer as it is, or a milky foundation (liquid cosmetic composition containing water) was applied. Thereafter, an excess foundation was removed. With respect to removal of the excess foundation, a cosmetic brush was used to remove the excess powder in case of the solid foundation, or in case of the milky foundation, the applied portion was pressed with tissue paper until the color was not transferred, thereby removing the excess milky lotion.

(b) Case of using water:

After the surface of the base layer was pressed for 1 minute with tissue paper wetted with water to make the base layer conformable to water, a solid foundation (powder) was applied, or a milky foundation (liquid cosmetic composition containing water) was applied. Thereafter, an excess foundation was removed. With respect to removal of the excess foundation, a cosmetic brush was used to remove the excess powder in case of the solid foundation, or in case of the milky foundation, the applied portion was pressed with tissue paper until the color was not transferred, thereby removing the excess milky lotion.

The color of the foundation applied to the base layer of the makeup-assisting patch was measured by a colorimeter (CM-3500d, manufactured by MINOLTA) to calculate a color difference from the woodfree paper. The result was ranked as AA (very good in spreading of the cosmetic composition) where the color difference from the woodfree paper was 15 or more, or A (good in spreading of the cosmetic composition) where the color difference from the woodfree paper was less than 15. The results are shown in Tables 2 and 3.

[Table 2]

| Solid foundation | | |
|---|---|---|
| | (a) Using no water | (b) Using water |
| Example 1 | A | AA |
| Example 2 | A | A |

[Table 3]

| Milky foundation | | |
|---|---|---|
| | (a) Using no water | (b) Using water |
| Example 1 | AA | AA |
| Example 2 | A | A |

[Consideration]

From the results shown in Tables 2 and 3, it was found that the spreading of the cosmetic composition is better in case (Example 1) of using the polyether type polyurethane elastomer as the base layer than case (Example 2) of using the polyester type polyurethane elastomer as the base layer.

[Example 3]

The makeup-assisting patch in Example 1 was used fitting to the form of damage or the like and stuck on subjects suffering from various diseases shown in Table 4 on their faces or arms, and a cosmetic composition was applied to make makeup. All the subjects made self-evaluation as to inconspicuousness, a feeling of physical disorder, a rash, touch and spreading of the cosmetic composition. As a result, they had good impressions in all the evaluation items and made evaluation to the effect that natural makeup can be made. From all the subjects, was obtained evaluation to the effect that (i) no peeling of the patch during sticking is found, (ii) no pain is felt upon peeling, (iii) peeling is easy, and (iv) the skin at the stuck portion is not swollen, or no sweat accumulates thereon after peeled. The fact that (i) no peeling of the patch during sticking is found indicates that the makeup-assisting patch according to the present invention has sufficient adhesion property. The fact that (ii) no pain is felt upon peeling indicates that the adhesion property of the makeup-assisting patch according to the present invention is not excessive. The fact that (iii) peeling is easy indicates that the strength of the makeup-assisting patch according to the present invention is sufficient. The fact that (iv) the skin at the stuck portion is not swollen, or no sweat accumulates thereon after peeled indicates that the water vapor permeability of the makeup-assisting patch according to the present invention is sufficiently high.
Thereafter, a VAS (visual analog scale) test as to the degree of satisfaction before and after makeup was conducted on all the subjects.
Time of evaluation: before makeup and after makeup
Evaluation method: Each subject has 100 points, and the subjects themselves judge by regarding the case where the appearance of a diseased part such as damage weighed on their mind as zero point, or the case where the appearance of a diseased part did not weigh on their mind as 100 points.

The results are shown in Table 4.

[Table 4]

| | Age | Condition of disease | Site | VAS | |
|---|---|---|---|---|---|
| | | | | Before makeup | After makeup |
| 1 | 24 | Scar from wrist cutting | Arm | 70 | 90 |
| 2 | 50 | Cleft lip, cleft palate | Face | 30 | 75 |
| 3 | 26 | External wound due to operation from traffic accident | Face | 20 | 100 |
| 4 | 56 | Scar from dog bite | Face | 5 | 95 |
| 5 | 51 | External wound from operation | Face | 30 | 100 |
| 6 | 35 | Scar after burn | Face | 0 | 100 |
| 7 | 26 | Scar from wrist cutting | Arm | 15 | 100 |
| 8 | 51 | External wound from operation | Face | 40 | 90 |
| 9 | 34 | External wound due to operation from traffic accident | Face | 20 | 90 |
| 10 | 39 | Left maxillar sinusitis, paralysis | Face | 60 | 65 |

[Consideration]

As apparent from the results shown in Table 4, most of the subjects are improved toward the direction that the appearance did not weigh on their mind by applying the makeup-assisting patch according to the present invention to the diseased part. This fact means the advantage of "receiving the final one's own appearance through makeup", which is a principal object of the makeup method intended for rehabilitation, can be achieved.

[Example 4]

The makeup-assisting patch in Example 1 was die-cut into a form of the drop (tear drop) illustrated in FIG. 1.

This piece was used to conduct a makeup method of pulling up pouched eyes in FIG. 3(1). As a result, the pouched eyes were pulled up.

[Example 5]

The makeup-assisting patch in Example 1 was die-cut into a form of the drop (tear drop) illustrated in FIG. 1. This piece was used to conduct a method of improving pouched cheeks in FIG. 3(4). As a result, the pouched cheeks looked tightened.

INDUSTRIAL APPLICABILITY

The makeup-assisting patches according to the present invention can conform to wrinkles and fine irregularities (fine furrows) of the skin, make a stuck portion inconspicuous, can easily follow the movement of the skin, are hard to cause a rash, can give the same touch as the healthy skin around them, protect the skin from cosmetic compositions and ultraviolet rays, and permit applying cosmetic compositions thereto in a stuck state. Therefore, the makeup-assisting patches according to the present invention are suitable for use as auxiliary materials in makeup methods intended for rehabilitation.

**Claims**

1.  A makeup-assisting patch having a layer structure **characterised in that** a pressure sensitive adhesive layer is provided on one surface of a base layer, wherein

    (1) the base layer is a layer of a polyether type polyurethane elastomer having a glass transition temperature within a range of -10 to -70°C,
    (2) the pressure sensitive adhesive layer is an acrylic pressure sensitive adhesive layer composed of a copolymer containing at least one monomer unit selected from the group consisting of an alkyl acrylate and an alkyl methacrylate each having an alkyl group having 8 to 12 carbon atoms in a proportion of 70% by weight or more,
    (3) the thickness of the base layer is 1 to 8 $\mu$m, the thickness of the pressure sensitive adhesive layer is 1 to 8 $\mu$m, and the total thickness of these both layers is 2 to 15 $\mu$m,
    (4) the pressure sensitive adhesive layer has a peeling force within a range of from 0.1 to 3 N/10 mm when a peeling force is measured at a crosshead speed of 300$\pm$30 mm/min in accordance with Japanese Industrial Standard JIS Z 0237,
    (5) the makeup-assisting patch has a tensile load within a range of from 0.01 to 1.2 N/cm in both machine and transverse directions when a 10% tensile load is measured in accordance with Japanese Industrial Standard JIS Z 0237,
    (6) the makeup-assisting patch has a water vapor transmission rate of 1,000 g/m2·24 hr or more when a water vapor transmission rate is measured under conditions of a temperature of 40°C and a relative humidity of 90% in accordance with Japanese Industrial Standard JIS Z 0208, and
    (7) the base layer is such that a surface provided with no pressure sensitive adhesive layer is subjected to embossing.

2.  The makeup-assisting patch according to claim 1, wherein the alkyl acrylate is at least one alkyl acrylate selected from the group consisting of 2-ethylhexyl acrylate, isooctyl acrylate, n-octyl acrylate and isononyl acrylate.

3.  The makeup-assisting patch according to claim 1, wherein the gloss of the embossed surface is adjusted within a range of from 0.5 to 7.5 as measured at an angle of 60°C.

4.  The makeup-assisting patch according to claim 1, wherein the base layer or the pressure sensitive adhesive layer or these both layers are colored yellow or orange with a colorant.

5.  The makeup-assisting patch according to claim 1, which is a tape or sheet and is die-cut into a form having a projected portion.

6.  The makeup-assisting patch according to claim 5, wherein the form having the projected portion is a drop form having a length ranging from 0.5 to 6 cm and a width ranging from 0.3 to 3 cm.

**7.** The makeup-assisting patch according to claim 1, wherein a carrier layer is additionally arranged on the surface of the base layer, and a separator layer is further arranged on the surface of the pressure sensitive adhesive layer, thereby having a 4 layer structure of carrier layer/base layer/pressure sensitive adhesive layer/separator layer.

**8.** A makeup method comprising sticking the makeup-assisting patch according to any one of claims 1 to 7 on the skin with the pressure-sensitive adhesive layer thereof.

**9.** The makeup method according to claim 8, wherein the makeup-assisting patch is stuck on the skin, and a cosmetic composition is then further applied to the surface of the skin including the surface of the base layer of the makeup-assisting patch stuck.

**10.** The makeup method according to claim 9, wherein the makeup-assisting patch is stuck on the skin, water is applied to the surface of the base layer of the makeup-assisting patch stuck make the surface conformable to water, and the cosmetic composition is then further applied to the surface of the skin including the surface of the base layer.

**11.** The makeup method according to claim 8, wherein the makeup-assisting patch is stuck on a wrinkled or pouched portion of the skin with the surface of the pressure sensitive adhesive layer thereof in a state that wrinkles or pouches have been stretched.

**12.** A method of producing a makeup-assisting patch according to claim 7, comprising forming a pressure sensitive adhesive layer on a separator layer, forming a polyurethane elastomer layer of a base layer on a carrier layer and then laminating the pressure sensitive adhesive layer on the separator layer and the polyurethane elastomer layer on the carrier layer on each other.

## Patentansprüche

**1.** Ein Make-up-Hilfspflaster mit einer Schichtstruktur, **dadurch gekennzeichnet, dass** eine druckempfindliche Klebeschicht auf einer Oberfläche einer Basisschicht bereitgestellt wird, wobei

(1) die Basisschicht eine Schicht eines polyetherartigen Polyurethanelastomers ist, die eine Glasübergangstemperatur in einem Bereich von -10 bis -70°C aufweist,
(2) die druckempfindliche Klebeschicht eine druckempfindliche Acrylklebeschicht ist, die aus einem Copolymer zusammengesetzt ist, das mindestens eine Monomereinheit ausgewählt aus der Gruppe, bestehend aus einem Alkylacrylat und einem Alkylmethacrylat, enthält, die jeweils eine Alkylgruppe mit 8 bis 12 Kohlenstoffatomen in einem Anteil von 70 Gewichts-% oder mehr aufweist,
(3) die Dicke der Basisschicht 1 bis 8 $\mu$m beträgt, die Dicke der druckempfindlichen Klebeschicht 1 bis 8 $\mu$m beträgt und die Gesamtdicke dieser beiden Schichten 2 bis 15 $\mu$m beträgt,
(4) die druckempfindliche Klebeschicht eine Schälkraft in einem Bereich von 0,1 bis 3 N/10 mm aufweist, wenn die Schälkraft bei einer Kreuzkopfgeschwindigkeit von 300 $\pm$ 30 mm/min gemäß der Japanischen Industrienorm JIS Z 0237 gemessen wird,
(5) das Make-up-Hilfspflaster eine Zugbelastung in einem Bereich von 0,01 bis 1,2 N/cm sowohl in Maschinen- als auch Querrichtung aufweist, wenn eine 10%ige Zugbelastung gemäß der Japanischen Industrienorm JIS Z 0237 gemessen wird,
(6) das Make-up-Hilfspflaster einen Wasserdampfdurchgangskoeffizient von 1000 $g/m^2\cdot24$ h oder mehr aufweist, wenn der Wasserdampfdurchgangskoeffizient unter den Bedingungen einer Temperatur von 40°C und einer relativen Luftfeuchtigkeit von 90% gemäß der Japanischen Industrienorm JIS Z 0208 gemessen wird, und
(7) die Basisschicht solcherart beschaffen ist, dass eine Oberfläche, die ohne druckempfindliche Klebeschicht bereitgestellt wird, einem Prägen unterzogen wird.

**2.** Das Make-up-Hilfspflaster gemäß Anspruch 1, wobei das Alkylacrylat mindestens ein Alkylacrylat ist, das aus der Gruppe ausgewählt ist, die aus 2-Ethylhexylacrylat, Isooctylacrylat, n-Octylacrylat und Isononylacrylat besteht.

**3.** Das Make-up-Hilfspflaster gemäß Anspruch 1, wobei der Glanz der geprägten Oberfläche in einem Bereich von 0,5 bis 7,5, gemessen bei einem Winkel von 60°C, angepasst wird.

**4.** Das Make-up-Hilfspflaster gemäß Anspruch 1, wobei die Basisschicht oder die druckempfindliche Klebeschicht oder diese beiden Schichten mit einem Färbemittel gelb oder orange gefärbt sind.

5. Das Make-up-Hilfspflaster gemäß Anspruch 1, das ein Band oder eine Platte ist und zu einer Form mit einem vorspringenden Teil gestanzt wird.

6. Das Make-up-Hilfspflaster gemäß Anspruch 5, wobei die Form mit dem vorspringenden Teil eine Tropfenform mit einer Länge im Bereich von 0,5 bis 6 cm und einer Breite im Bereich von 0,3 bis 3 cm ist.

7. Das Make-up-Hilfspflaster gemäß Anspruch 1, wobei eine Trägerschicht zusätzlich auf der Oberfläche der Basisschicht angeordnet ist und ferner eine Trennschicht auf der Oberfläche der druckempfindlichen Klebeschicht angeordnet ist, so dass es eine 4-Schichtenstruktur aus Trägerschicht/Basisschicht/druckempfindlicher Klebeschicht/Trennschicht aufweist.

8. Ein Make-up-Verfahren, das das Aufkleben des Make-up-Hilfspflasters gemäß einem der Ansprüche 1 bis 7 mit der druckempfindlichen Klebeschicht davon auf die Haut beinhaltet.

9. Das Make-up-Verfahren gemäß Anspruch 8, wobei das Make-up-Hilfspflaster auf die Haut aufgeklebt wird und dann ferner eine kosmetische Zusammensetzung auf die Hautoberfläche aufgebracht wird, einschließlich der Oberfläche der Basisschicht des aufgeklebten Make-up-Hilfspflasters.

10. Das Make-up-Verfahren gemäß Anspruch 9, wobei das Make-up-Hilfspflaster auf die Haut aufgeklebt wird, Wasser auf die Oberfläche der Basisschicht des aufgeklebten Make-up-Hilfspflaster aufgebracht wird, um die Oberfläche mit Wasser vereinbar zu machen, und dann ferner eine kosmetische Zusammensetzung auf die Hautoberfläche einschließlich der Oberfläche der Basisschicht aufgebracht wird.

11. Das Make-up-Verfahren gemäß Anspruch 8, wobei das Make-up-Hilfspflaster mit der Oberfläche der druckempfindlichen Klebeschicht davon auf einen runzligen oder schlaffen Teil der Haut in einem solchen Zustand aufgebracht wird, dass die Runzeln oder die schlaffe Haut gedehnt worden sind.

12. Ein Verfahren zum Herstellen eines Make-up-Hilfspflasters gemäß Anspruch 7, das Folgendes beinhaltet: Bilden einer druckempfindlichen Klebeschicht auf einer Trennschicht, Bilden einer Polyurethanelastomerschicht einer Basisschicht auf einer Trägerschicht und anschließendes Laminieren der druckempfindlichen Klebeschicht auf der Trennschicht und der Polyurethanelastomerschicht auf der Trägerschicht aufeinander.

**Revendications**

1. Timbre d'aide au maquillage ayant une structure en couches **caractérisée en ce qu'**une couche adhésive sensible à la pression est fournie sur une surface d'une couche de base, dans lequel

(1) la couche de base est une couche en un élastomère de polyuréthane du type polyéther ayant une température de transition vitreuse dans une plage de -10 à -70 °C,
(2) la couche adhésive sensible à la pression est une couche adhésive sensible à la pression en acrylique composée d'un copolymère contenant au moins une unité monomère sélectionnée parmi le groupe consistant en un acrylate d'alkyle et un méthacrylate d'alkyle ayant chacun un groupe alkyle ayant 8 à 12 atomes de carbone en une proportion de 70 % en poids ou plus,
(3) l'épaisseur de la couche de base est de 1 à 8 $\mu$m, l'épaisseur de la couche adhésive sensible à la pression est de 1 à 8 $\mu$m, et l'épaisseur totale de ces deux couches est de 2 à 15 $\mu$m,
(4) la couche adhésive sensible à la pression a une force de pelage dans une plage de 0,1 à 3 N/10 mm lorsqu'une force de pelage est mesurée à une vitesse de traverse de 300±30 mm/min conformément à la norme industrielle japonaise JIS Z 0237,
(5) le timbre d'aide au maquillage a une contrainte de traction dans une plage de 0,01 à 1,2 N/cm dans la direction machine et transversale toutes les deux lorsqu'une contrainte de traction de 10 % est mesurée conformément à la norme industrielle japonaise JIS Z 0237,
(6) le timbre d'aide au maquillage a un taux de transmission de vapeur d'eau de 1000 g/m$^2$·24 h ou plus lorsqu'un taux de transmission de vapeur d'eau est mesuré dans des conditions d'une température de 40 °C et d'une humidité relative de 90 % conformément à la norme industrielle japonaise JIS Z 0208, et
(7) la couche de base est telle qu'une surface fournie sans couche adhésive sensible à la pression est soumise au gaufrage.

**2.** Timbre d'aide au maquillage selon la revendication 1, dans lequel l'acrylate d'alkyle est au moins un acrylate d'alkyle sélectionné parmi le groupe consistant en acrylate de 2-éthylhexyle, acrylate d'isooctyle, acrylate de n-octyle et acrylate d'isononyle.

**3.** Timbre d'aide au maquillage selon la revendication 1, dans lequel le lustre de la surface gaufrée est ajusté dans une plage de 0,5 à 7,5 tel que mesuré à un angle de 60 °C.

**4.** Timbre d'aide au maquillage selon la revendication 1, dans lequel la couche de base ou la couche adhésive sensible à la pression ou ces deux couches sont colorées en jaune ou orange avec un colorant.

**5.** Timbre d'aide au maquillage selon la revendication 1, qui est un ruban ou une feuille et qui est découpé à l'emporte-pièce en une forme ayant une partie en saillie.

**6.** Timbre d'aide au maquillage selon la revendication 5, dans lequel la forme ayant une partie en saillie est une forme de goutte ayant une longueur allant de 0,5 à 6 cm et une largeur allant de 0,3 à 3 cm.

**7.** Timbre d'aide au maquillage selon la revendication 1, dans lequel une couche porteuse est arrangée en plus sur la surface de la couche de base, et une couche de séparation est arrangée en outre sur la surface de la couche adhésive sensible à la pression, ayant ainsi une structure en 4 couches de couche porteuse/couche de base/couche adhésive sensible à la pression/couche de séparation.

**8.** Méthode de maquillage comprenant coller le timbre d'aide au maquillage selon l'une quelconque des revendications 1 à 7, sur la peau avec la couche adhésive sensible à la pression de celui-ci.

**9.** Méthode de maquillage selon la revendication 8, dans laquelle le timbre d'aide au maquillage est collé sur la peau, et une composition cosmétique est alors appliquée en outre à la surface de la peau incluant la surface de la couche de base du timbre d'aide au maquillage collé.

**10.** Méthode de maquillage selon la revendication 9, dans laquelle le timbre d'aide au maquillage est collé sur la peau, de l'eau est appliquée à la surface de la couche de base du timbre d'aide au maquillage collé rendant la surface façonnable à l'eau, et la composition cosmétique est alors appliquée en plus à la surface de la peau incluant la surface de la couche de base.

**11.** Méthode de maquillage selon la revendication 8, dans laquelle le timbre d'aide au maquillage est collé sur une partie ridée ou bouffie de la peau avec la surface de la couche adhésive sensible à la pression de celui-ci dans un état dans lequel les rides ou les poches ont été tirées.

**12.** Procédé de fabrication d'un timbre d'aide au maquillage selon la revendication 7, comprenant former une couche adhésive sensible à la pression sur une couche de séparation, former une couche en élastomère de polyuréthane d'une couche de base sur une couche porteuse et puis stratifier la couche adhésive sensible à la pression sur la couche de séparation et la couche en élastomère de polyuréthane sur la couche porteuse l'une sur l'autre.

[Fig. 1]

[Fig. 2]

[Fig. 3]

(1)

(2)

(3)

(4)

(5)

(6)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3116047 B
- JP 10194962 A
- JP 2004313277 A
- JP NO10234772 B
- JP 55001747 A
- JP 56104519 A
- JP 10234772 A